# EUROPEAN PATENT APPLICATION

(11) **EP 3 418 374 A1**
(43) Date of publication of application: **26.12.2018**
(21) Application number: 17753157.1
(22) Date of filing: 14.02.2017
(51) Int. Cl.: C12N 1/00, C12N 5/0797

(54) **CULTURE MEDIUM FOR USE IN DIFFERENTIATION OF PLURIPOTENT STEM CELL INTO NEURAL STEM CELL, AND USE THEREOF**

(30) Priority: 16.02.2016 JP 2016027324
(71) Applicant: Keio University, Tokyo 108-8345 (JP)
(72) Inventor: OKANO, Hideyuki, Tokyo 160-8582 (JP); AKAMATSU, Wado, Tokyo 160-8582 (JP); FUJIMORI, Koki, Tokyo 160-8582 (JP); NODA, Tomoko, Tokyo 160-8582 (JP); ANDOH, Takayuki, Tokyo 160-8582 (JP); TEZUKA, Toshiki, Tokyo 160-8582 (JP); MATSUMOTO, Takuya, Tokyo 160-8582 (JP)
(74) Representative: HGF Limited
(86) International application number: PCT/JP2017/005291
(87) International publication number: WO 2017/141900

(57) **Abstract**

A method is provided for uniformly differentiating a pluripotent stem cell into a neural stem cell, with elimination of variation among cell strains or clones of the pluripotent stem cell and without formation of an embryoid body, regardless of the origin of the pluripotent stem cell

## Description

### Technical Field

The present invention relates to a culture medium for use in differentiation of a pluripotent stem cell into a neural stem cell and a use thereof. More specifically, the present invention relates to a method for differentiating a pluripotent stem cell into a neural stem cell, a method for improving the efficiency of differentiating a pluripotent stem cell into a neural stem cell, a culture medium for use in differentiation of a pluripotent stem cell into a neural stem cell, a method for producing a neural stem cell, and a neural stem cell. Priority is claimed on Japanese Patent Application No. 2016-027324, filed on February 16, 2016, the content of which is incorporated herein by reference.

### Background Art

Studies of neurological diseases have been carried out mainly using animal models and immortalized neuronal cell lines because of the difficulty of analyzing the central nervous system of patients. With the recent advancement of induced pluripotent stem cell (hereinafter, sometimes referred to as "iPSC") technology, it has become possible to utilize a patient-specific cultured nerve cell as a neurological disease model.

Patient-specific iPSCs have been conventionally produced mainly from dermal fibroblasts. However, in order to obtain a dermal fibroblast cell line derived from a patient, it is necessary to harvest the skin, which involves problems such as bleeding, infections, and scars. For this reason, it is preferable to produce patient-specific iPSCs by a less-invasive technique.

Therefore, a technology for producing iPSCs from peripheral blood cells that can be harvested by a less-invasive technique has been studied. For example, it has been reported that CD3-positive T cells can be effectively reprogrammed into iPSCs using a Sendai virus vector (see, for example, NPL 1).

### Citation List

### Non-Patent Literature

[NPL 1] Seki T., et al., Generation of induced pluripotent stem cells from human terminally differentiated circulating T cells., Cell Stem Cell, 7 (1), 11-14, 2010.

### Summary of Invention

### Technical Problem

A CD3-positive T cell can be cultured in the presence of recombinant interleukin (IL)-2 on a plate coated with anti-CD3 monoclonal antibodies and can be stored frozen. For this reason, a CD3-positive T cell is ideal for producing patient-specific iPSCs.

However, the present inventors have found that, upon comparing with dermal fibroblast-derived iPSCs, it is difficult to achieve the differentiation of T cell-derived iPSCs into a nervous system in a conventional differentiation induction method through the formation of an embryoid body (EB).

In addition, the degree of residual epigenetic memory varies depending on the strain of iPS cells. Therefore, it has been difficult to differentiate a plurality of iPS cell lines into neural stem cells, and even nerve cells with the same efficiency using a conventional neural differentiation induction method. In particular, in blood cell-derived iPS cells, for which residual epigenetic memory is the main factor in neural differentiation resistance, it has been difficult to equalize differentiation induction efficiency among strains of iPS cells.

Therefore, an object of the present invention is to provide a technique for efficiently inducing the differentiation of a pluripotent stem cell into a neural stem cell in a short period of time, with elimination of variation in the induction efficiency among cell strains of the pluripotent stem cell and without going through an embryoid body. Further, another object of the present invention is to provide a technique for efficiently inducing differentiation into a neural stem cell also from a blood cell-derived pluripotent stem cell in a short period of time without going through an embryoid body.

### Solution to Problem

The present invention includes the following aspects.
[1] A method for uniformly differentiating a pluripotent stem cell into a neural stem cell, with elimination of variation among cell strains or clones of the pluripotent stem cell and without formation of an embryoid body, regardless of the origin of the pluripotent stem cell.
[2] The method according to [1], including culturing the pluripotent stem cell in a culture medium containing a fibroblast growth factor 2 (FGF 2), a rho-associated protein kinase (ROCK) inhibitor, and a leukemia inhibitory factor (LIF) as active components.
[3] The method according to [2], in which the culturing is carried out under hypoxic conditions.
[4] The method according to [2] or [3], further including dissociating the pluripotent stem cells into individual cells one by one before the culturing.
[5] The method according to any one of [1] to [4], in which the pluripotent stem cell is a blood cell-derived induced pluripotent stem cell.
[6] The method according to [5], in which the induced pluripotent stem cell is a T cell-derived cell.
[7] A culture medium containing FGF2, a ROCK inhibitor, and LIF as active components, for use in the method according to any one of [1] to [6].
[8] A neural stem cell produced by the method according to any one of [1] to [6].
[9] The neural stem cell according to [8], which expresses a forebrain marker and a forebrain/midbrain marker.
[10] The neural stem cell according to [8] or [9], which does not substantially express homeobox B4 (HOXB 4).
[11] The neural stem cell according to any one of [8] to [10], in which a T cell receptor gene is rearranged.
[12] A method for improving the efficiency of differentiating a pluripotent stem cell into a neural stem cell, the method including culturing the pluripotent stem cell in a culture medium containing FGF2, a ROCK inhibitor, and LIF as active components.
[13] The method according to [12], in which the improved efficiency of differentiating a pluripotent stem cell into a neural stem cell is the efficiency equivalent to that of differentiating a dermal fibroblast-derived induced pluripotent stem cell into a neural stem cell.
[14] The method according to [12] or [13], in which the culturing is carried out under hypoxic conditions.
[15] The method according to any one of [12] to [14], further including dissociating the pluripotent stem cells into individual cells one by one before the culturing.
[16] The method according to any one of [12] to [15], in which the pluripotent stem cell is a blood cell-derived induced pluripotent stem cell.
[17] The method according to [16], in which the induced pluripotent stem cell is a T cell-derived cell.
[18] A culture medium containing FGF2, a ROCK inhibitor, and LIF as active components, for use in the method according to any one of [12] to [17].

[A1] A culture medium for use in differentiation of a pluripotent stem cell into a neural stem cell, which contains FGF2, a ROCK inhibitor, and LIF as active components.
[A2] The culture medium for use in differentiation according to [A1], in which the pluripotent stem cell is an induced pluripotent stem cell.
[A3] A method for producing a neural stem cell, including culturing a pluripotent stem cell in the culture medium according to [A1] or [A2].
[A4] The production method according to [A3], in which the pluripotent stem cell is an induced pluripotent stem cell.
[A5] The production method according to [A3] or [A4], in which the culturing is carried out under a hypoxic environment.
[A6] The production method according to any one of [A3] to [A5], further including dissociating the pluripotent stem cells into individual cells one by one before the culturing.
[A7] A neural stem cell which does not substantially express HOXB4.
[A8] The neural stem cell according to [A7], in which a T cell receptor gene is rearranged.

### Advantageous Effects of Invention

According to the present invention, it is possible to provide a technique for inducing the differentiation of a pluripotent stem cell into a neural stem cell easily and efficiently in a short period of time without going through an embryoid body.

### Brief Description of Drawings

FIG. 1 is a diagram showing a procedure of differentiation of iPSCs into neural stem cells by a conventional method.
FIG. 2(a) is a representative photograph of a neural stem cell mass formed from adult human dermal fibroblast-derived iPSC (aHDF-iPSC) in Experimental Example 2. FIG. 2(b) is a representative photograph of a neural stem cell mass formed from TiPSC in Experimental Example 2.
FIG. 3 is a graph showing the results of the measurement of the number of neural stem cell masses formed in Experimental Example 2.
FIG. 4 is a diagram showing a procedure of differentiation of iPSCs into neural stem cells by a novel method.
FIG. 5(a) is a representative photograph of a neural stem cell mass formed from aHDF-iPSC in Experimental Example 3. FIG. 5(b) is a representative photograph of a neural stem cell mass formed from T cell-derived iPSC (TiPSC) in Experimental Example 3.
FIG. 6 is a graph showing the results of the measurement of the number of neural stem cell masses formed in Experimental Example 3.
FIG. 7 is a graph showing the results of the measurement of the number of neural stem cell masses formed in Experimental Example 4.

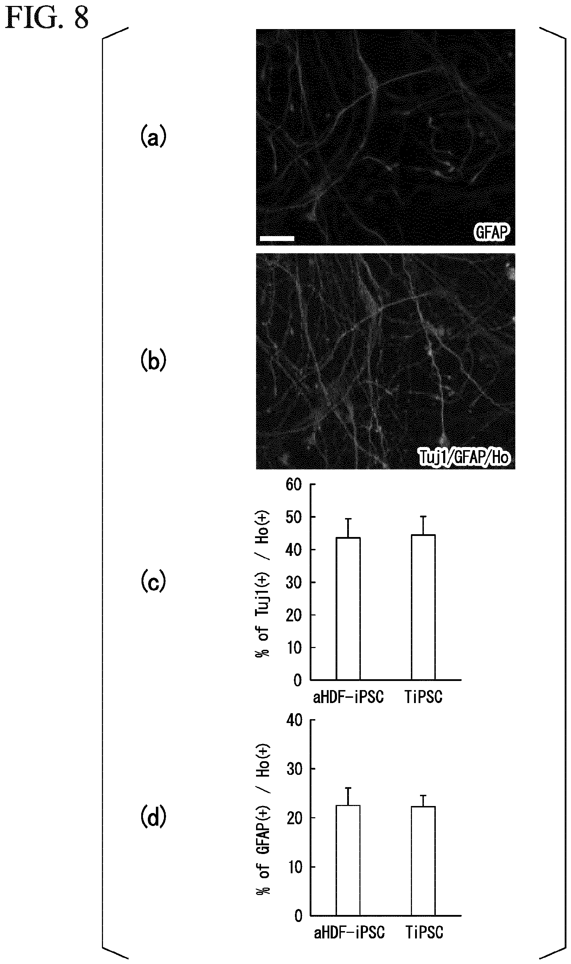

FIG. 8(a) is a representative photograph showing the results of the differentiation of the TiPSC-derived neural stem cell masses in Experimental Example 5, followed by fluorescent immunostaining with an antibody against GFAP, which is an astrocyte marker. FIG. 8(b) is a photograph showing the results of further staining the cells in the same field of view as in FIG. 8(a) with an antibody against Tuj1, which is a neuronal marker, and Hoechst 33342. FIG. 8(c) is a graph showing the results of the measurement of the ratio of anti-Tuj1 antibody-stained cells to Hoechst 33342-stained cells, in cells differentiated from the TiPSC-derived neural stem cell mass and cells differentiated from the aHDF-iPSC-derived neural stem cell mass. FIG. 8(d) is a graph showing the results of the measurement of the ratio of anti-GFAP antibody-stained cells to Hoechst 33342-stained cells, in cells differentiated from the TiPSC-derived neural stem cell mass and cells differentiated from the aHDF-iPSC-derived neural stem cell mass.

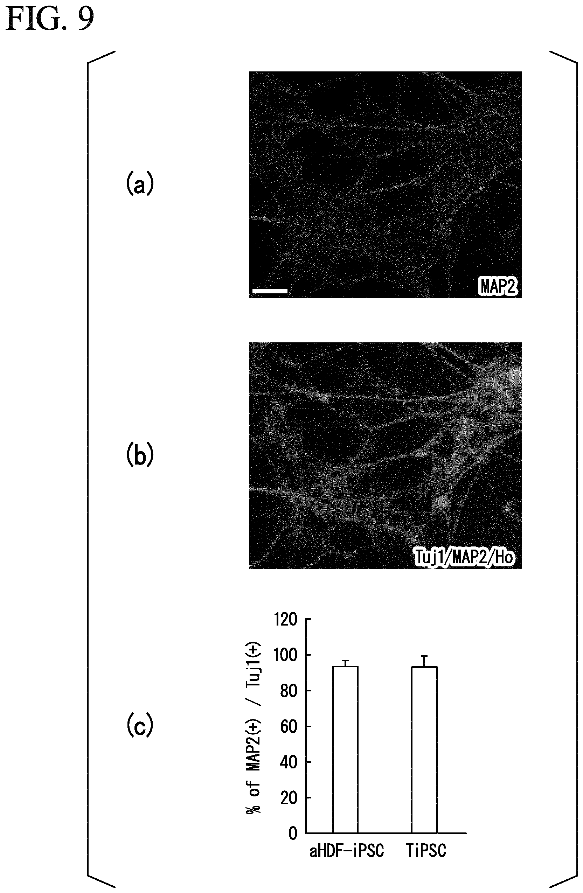

FIG. 9(a) is a representative photograph showing the results of the differentiation of the TiPSC-derived neural stem cell mass in Experimental Example 5, followed by fluorescent immunostaining with an antibody against MAP2, which is a neuronal marker. FIG. 9(b) is a photograph showing the results of further staining the cells in the same field of view as in FIG. 9(a) with an antibody against Tuj1, which is another neuronal marker, and Hoechst 33342. FIG. 9(c) is a graph showing the results of the measurement of the ratio of anti-MAP2 antibody-stained cells to anti-Tuj 1 antibody-stained cells, in cells differentiated from the TiPSC-derived neural stem cell mass and cells differentiated from the aHDF-iPSC-derived neural stem cell mass.

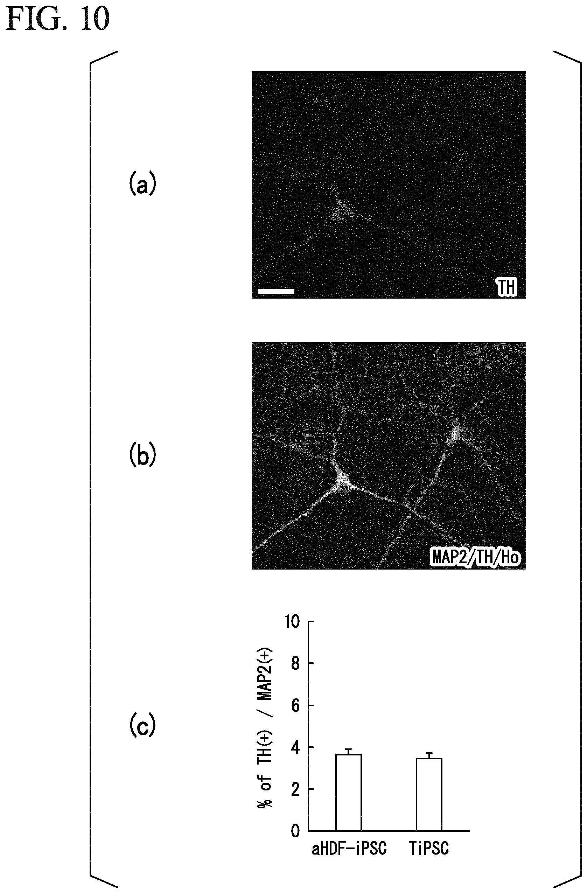

FIG. 10(a) is a representative photograph showing the results of the differentiation of the TiPSC-derived neural stem cell mass in Experimental Example 5, followed by fluorescent immunostaining with an antibody against tyrosine hydroxylase (TH), which is a dopaminergic neuronal marker. FIG. 10(b) is a photograph showing the results of further staining the cells in the same field of view as in FIG. 10(a) with an antibody against MAP2, which is a neuronal marker, and Hoechst 33342. FIG. 10(c) is a graph showing the results of the measurement of the ratio of anti-TH antibody-stained cells to anti-MAP2 antibody-stained cells, in cells differentiated from the TiPSC-derived neural stem cell mass and cells differentiated from the aHDF-iPSC-derived neural stem cell mass.

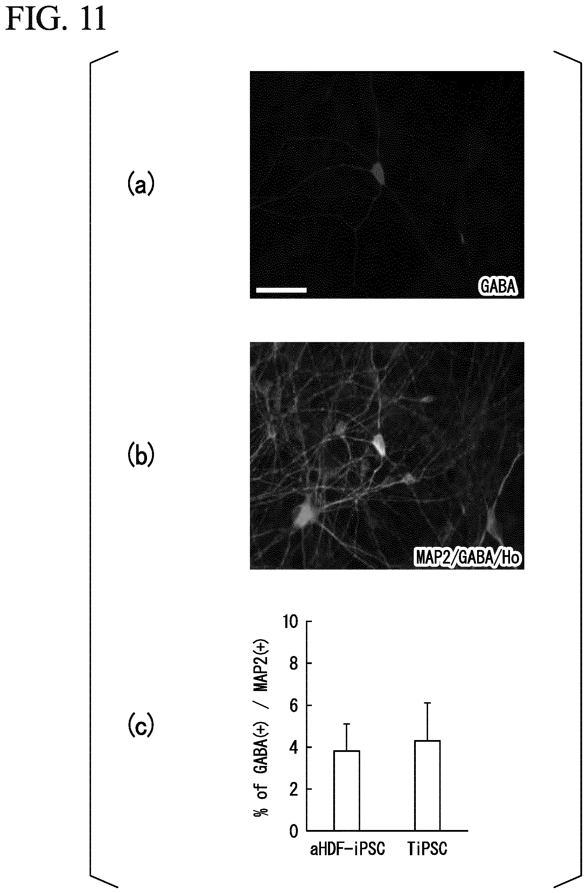

FIG. 11(a) is a representative photograph showing the results of the differentiation of the TiPSC-derived neural stem cell mass in Experimental Example 5, followed by fluorescent immunostaining with an antibody against γ-aminobutyric acid (GABA). FIG. 11(b) is a photograph showing the results of further staining the cells in the same field of view as in FIG. 11(a) with an antibody against MAP2, which is a neuronal marker and Hoechst 33342. FIG. 11(c) is a graph showing the results of the measurement of the ratio of anti-GABA antibody-stained cells to anti-MAP2 antibody-stained cells, in cells differentiated from the TiPSC-derived neural stem cell mass and cells differentiated from the aHDF-iPSC-derived neural stem cell mass.

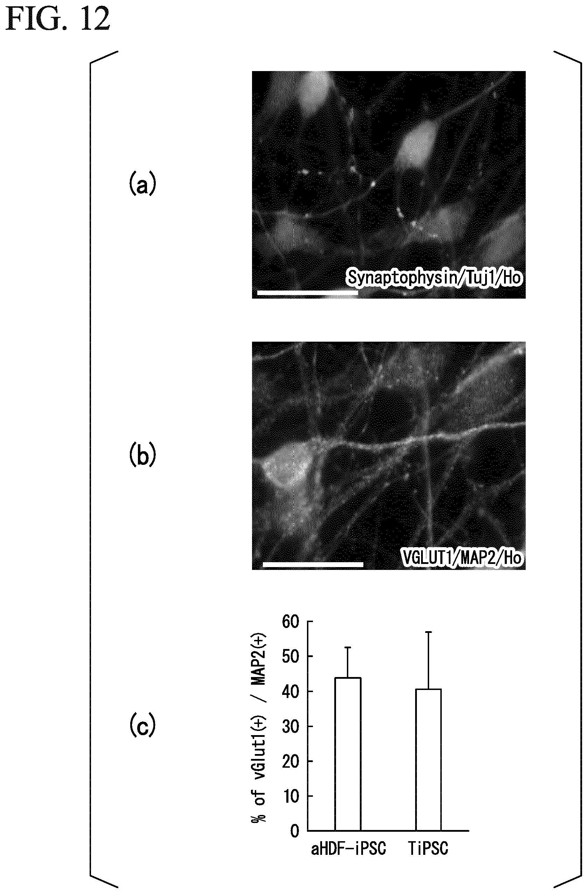

FIG. 12(a) is a representative photograph showing the results of the differentiation of the TiPSC-derived neural stem cell mass in Experimental Example 5, followed by staining with an anti-synaptophysin antibody, an anti-MAP2 antibody, and Hoechst 33342. FIG. 12(b) is a photograph showing the results of staining the cells in the same field of view as in FIG. 12(a) with an antibody against vesicular glutamate transporter 1 (vGLUT1), an anti-MAP2 antibody, and Hoechst 33342. FIG. 12(c) is a graph showing the results of the measurement of the ratio of anti-vGLUT1 antibody-stained cells to anti-MAP2 antibody-stained cells, in cells differentiated from the TiPSC-derived neural stem cell mass and cells differentiated from the aHDF-iPSC-derived neural stem cell mass.

### Description of Embodiments

### [Method of differentiating pluripotent stem cell directly into neural stem cell]

In one embodiment, the present invention provides a method for uniformly differentiating a pluripotent stem cell into a neural stem cell, with elimination of variation among cell strains or clones of the pluripotent stem cell and without formation of an embryoid body, regardless of the origin of the pluripotent stem cell.

In the method of the present embodiment, the pluripotent stem cell may be a pluripotent stem cell derived from a living body or an induced pluripotent stem cell. In the present specification, the phrase "regardless of the origin of the pluripotent stem cell" means that a pluripotent stem cell can be differentiated into a neural stem cell, regardless of a tissue from which the pluripotent stem cell is derived, a method of establishing an induced pluripotent stem cell, a vector used for establishing an induced pluripotent stem cell, a method of culturing an (induced) pluripotent stem cell, or the like.

In addition, the expression "uniformly differentiating a pluripotent stem cell into a neural stem cell, with elimination of variation among cell strains or clones of the pluripotent stem cell" means that a pluripotent stem cell can be differentiated into a neural stem cell with comparable efficiency, even in the case where there is variation in the degree of residual epigenetic memory or the like among cell strains or clones of the pluripotent stem cell.

Conventionally, the differentiation efficiency into a neural stem cell was sometimes low depending on the origin of the pluripotent stem cell. On the other hand, as will be described later in the Examples, according to the method of the present embodiment, it is possible to uniformly differentiate a pluripotent stem cell into a neural stem cell with elimination of variation among strains or clones of the pluripotent stem cell, regardless of the origin of the pluripotent stem cell.

As will be described later, the method of the present embodiment preferably includes a step of culturing a pluripotent stem cell in a culture medium containing FGF2, a ROCK inhibitor, and LIF as active components.

### [Method for improving efficiency of differentiating pluripotent stem cell into neural stem cell]

In one embodiment, the present invention provides a method for improving the efficiency of differentiating a pluripotent stem cell into a neural stem cell, including a step of culturing the pluripotent stem cell in a culture medium containing FGF2, a ROCK inhibitor, and LIF as active components.

It is known that dermal fibroblast-derived induced pluripotent stem cells have a relatively high efficiency of differentiation into neural stem cells. In contrast, conventionally, the differentiation efficiency into neural stem cells was sometimes low depending on the origin of the pluripotent stem cell.

As will be described later in the Examples, according to the method of the present embodiment, even in the case where pluripotent stem cells are pluripotent stem cells other than dermal fibroblast-derived induced pluripotent stem cells, the efficiency of differentiating pluripotent stem cells into neural stem cells can be improved to the same efficiency as the efficiency of differentiating dermal fibroblast-derived induced pluripotent stem cells into neural stem cells.

Further, according to the method of the present embodiment, the differentiation efficiency of dermal fibroblast-derived induced pluripotent stem cells into neural stem cells can also be improved, as compared with a conventional method of forming an embryoid body to differentiate pluripotent stem cells into neural stem cells.

As will be described later, the method of the present embodiment preferably includes a step of culturing a pluripotent stem cell in a culture medium containing FGF2, a ROCK inhibitor, and LIF as active components.

### [Culture medium for use in differentiation]

In one embodiment, the present invention provides a culture medium for use in differentiation of a pluripotent stem cell into a neural stem cell, which contains FGF2, a ROCK inhibitor, and LIF as active components.

As will be described later in the Examples, according to the culture medium of the present embodiment, pluripotent stem cells can be efficiently differentiated into neural stem cells. More specifically, by culturing the cells in the culture medium of the present embodiment, T-cell-derived iPSC (hereinafter, sometimes referred to as "TiPSC"), which is difficult to differentiate into the nervous system by the conventional method, can be differentiated into a neural stem cell with the same efficiency as that of differentiation of adult human dermal fibroblast-derived iPSC (hereinafter, sometimes referred to as "aHDF-iPSC") into a neural stem cell. In addition, the present invention can be applied to an immortalized lymphoblastoid cell line.

For this reason, for example, cells of the nervous system can be produced using iPSCs derived from peripheral blood cells that can be harvested with less invasiveness, and can be used as a neurological disease model.

The culture medium of the present embodiment can be used for the purpose of uniformly differentiating pluripotent stem cells directly into neural stem cells without forming an embryoid body regardless of the origin of pluripotent stem cell. Alternatively, the culture medium of the present embodiment can be used for the purpose of improving the efficiency of differentiating pluripotent stem cells (excluding dermal fibroblast-derived induced pluripotent stem cells) into neural stem cells.

In the culture medium of the present embodiment, in the case where the pluripotent stem cell to be differentiated is a human-derived cell, FGF2 and LIF are preferably derived from a human, and the ROCK inhibitor is also preferably one targeting human ROCK. In addition, for example, in the case where the pluripotent stem cell to be differentiated is a mouse-derived cell, FGF2 and LIF are preferably derived from a mouse, and the ROCK inhibitor is also preferably one targeting mouse ROCK.

The RefSeq ID of the human FGF2 protein is NP_001997, and the RefSeq ID of the mouse FGF2 protein is NP_032032. In addition, the RefSeq ID of the human LIF protein is NP_001244064, and the RefSeq ID of the mouse LIF protein is NP_001034626.

The ROCK inhibitor may be, for example, Y27632.

In the culture medium of the present embodiment, the pluripotent stem cell to be differentiated may be, for example, an ES cell, and may be, for example, an induced pluripotent stem cell (iPSC).

In the case where pluripotent stem cells are differentiated into neural stem cells in the culture medium of the present embodiment, the neural stem cells form neural stem cell mass (neurosphere). The formed neural stem cell mass can be subcultured in the state of neural stem cells by dissociating the mass into individual cells one by one and culturing the cells again in the culture medium of the present embodiment.

The culture medium of the present embodiment may be supplied as a liquid or as a powder. In addition, FGF2, ROCK inhibitor, or LIF may be supplied in a separate container and added to the culture medium at the time of use.

### [Method for producing neural stem cell]

In one embodiment, the present invention provides a method for producing a neural stem cell, including a step of culturing a pluripotent stem cell in above-mentioned culture medium.

In the production method of the present embodiment, the pluripotent stem cell to be differentiated may be, for example, an ES cell, and may be, for example, an induced pluripotent stem cell (iPSC).

As will be described later in the Examples, neural stem cells can be produced by culturing pluripotent stem cells in the above-mentioned culture medium. In the case where neural stem cells are produced from pluripotent stem cells, the conventional differentiation induction method through the formation of an embryoid body (EB) requires about 1.5 months. In contrast, according to the production method of the present embodiment, it is possible to differentiate pluripotent stem cells into neural stem cells in about 14 days.

Further, according to the production method of the present embodiment, neural stem cells can be produced by differentiating TiPSC, which is difficult to differentiate into the nervous system by the conventional method, into a neural stem cell with the same efficiency as that of differentiation of aHDF-iPSC into a neural stem cell.

In the production method of the present embodiment, the step of culturing pluripotent stem cells in the above-mentioned culture medium is preferably carried out under a hypoxic environment. The hypoxic environment may be, for example, an environment in which the oxygen concentration is 1 to 10% (v/v), for example, 1 to 5% (v/v). As will be described later in the Examples, the step of culturing pluripotent stem cells in the above-mentioned culture medium is carried out in a hypoxic environment, whereby the number of pluripotent stem cells to be produced can be increased.

Further, the production method of the present embodiment preferably further includes a step of dissociating pluripotent stem cells into individual cells one by one before the step of culturing the pluripotent stem cells in the above-mentioned culture medium. By dissociating pluripotent stem cells into individual cells one by one, the number of pluripotent stem cells to be produced can be increased.

### [Neural stem cell]

In one embodiment, the present invention provides a neural stem cell that does not substantially express HOXB4. The neural stem cell of the present embodiment can be produced by the above-mentioned method for producing a neural stem cell.

As will be described later in the Examples, it was revealed that the neural stem cells produced by the above-mentioned method for producing a neural stem cell hardly expressed HOXB4, which is a marker expressed in the myelencephalon and spinal cord. That is, substantially not expressing HOXB4 indicates that the cell is a neural stem cell produced by the above-mentioned method for producing a neural stem cell. Here, the phrase "does not substantially express" means that almost no expression of HOXB4 can be detected in the case of being confirmed by fluorescent immunostaining.

In addition, the RefSeq ID of the human HOXB4 protein is NP_076920, and the RefSeq ID of the mouse HOXB4 protein is NP_034589.

In one embodiment, the neural stem cell described above may have a rearrangement of the T cell receptor gene. The rearrangement of the T cell receptor gene indicates that this neural stem cell is derived from a mature T cell.

That is, the T cell receptor gene is rearranged in the neural stem cell produced by the above-mentioned method for producing a neural stem cell, using TiPSC produced from a mature T cell as a material. In addition, as described above, this neural stem cell is characterized in that it does not substantially express HOXB4.

### EXAMPLES

Hereinafter, the present invention will be described in more detail with reference to Examples, but the present invention is not limited to the following Examples.

### [Experimental Example 1]

### (Production of iPSC from T cell and dermal fibroblast)

iPSCs were produced from T cells and dermal fibroblasts harvested from a healthy subject. First, an episomal vector containing OCT4, SOX2, KLF4, L-MYC, LIN28, EBNA1, and shRNA against p53 or dominant negative p53 was introduced into CD3-positive lymphocytes derived from a healthy subject, whereby human T cell-derived iPSC (T-cell-derived iPSC, hereinafter sometimes referred to as "TiPSC") strains eTKA4 and eTKA5 were established.

Sendai virus vectors (model name "CytoTune (trademark)", available from DNAVEC Corporation) containing OCT4, SOX2, KLF4, and c-MYC in four vectors, respectively, were introduced into CD3-positive lymphocytes derived from a healthy subject, whereby TiPSC strains TKA7 and TKA14 were established.

In addition, a Sendai virus vector (manufactured by AIST) containing OCT4, SOX2, KLF4, and c-MYC in a single vector was introduced into CD3-positive lymphocytes derived from a healthy subject, whereby TiPSC strains TKA4 and TKA9 were established.

In addition, a retrovirus expressing OCT4, SOX2, KLF4, and c-MYC was introduced into dermal fibroblasts derived from a healthy subject, whereby adult human dermal fibroblast-derived iPSC (hereinafter, sometimes referred to as "aHDF- iPSC") strains KA11 and KA23 were established.

In addition, the above-mentioned episomal vector was introduced into dermal fibroblasts derived from a healthy subject, whereby aHDF-iPSC strains eKA3 and eKA4 were established.

Immunochemical analysis confirmed that all established TiPSC strains expressed pluripotency markers TRA-1-60 and stage-specific embryonic antigen 4 (SSEA4) at the same level as the established aHDF-iPSC strains. In addition, the transgene used for reprogramming was not detected by RT-PCR. In addition, as a result of analysis using a comparative genomic hybridization array, no significant difference in genomic structure was observed between TiPSC and aHDF-iPSC. In addition, as a result of examining the rearrangement of the T cell receptor gene by PCR, the rearrangement was not observed in aHDF-iPSC, whereas the rearrangement was confirmed in TiPSC.

### <In the conventional method through formation of embryoid body (EB), TiPSC was less likely to differentiate into the nervous system as compared to aHDF-iPSC>

### [Experimental Example 2]

### (Differentiation of iPSC into neural stem cell by conventional method)

The present inventors differentiated plural types of aHDF-iPSC strains and plural types of TiPSC strains into neural stem cells by the conventional EB method and DSi-EB method. As the aHDF-iPSC strain, the above-mentioned KA11, KA23, eKA3, and eKA4 were used. As the TiPSC strain, the above-mentioned eTKA4, eTKA5, TKA7, TKA14, TKA4, and TKA9 were used.

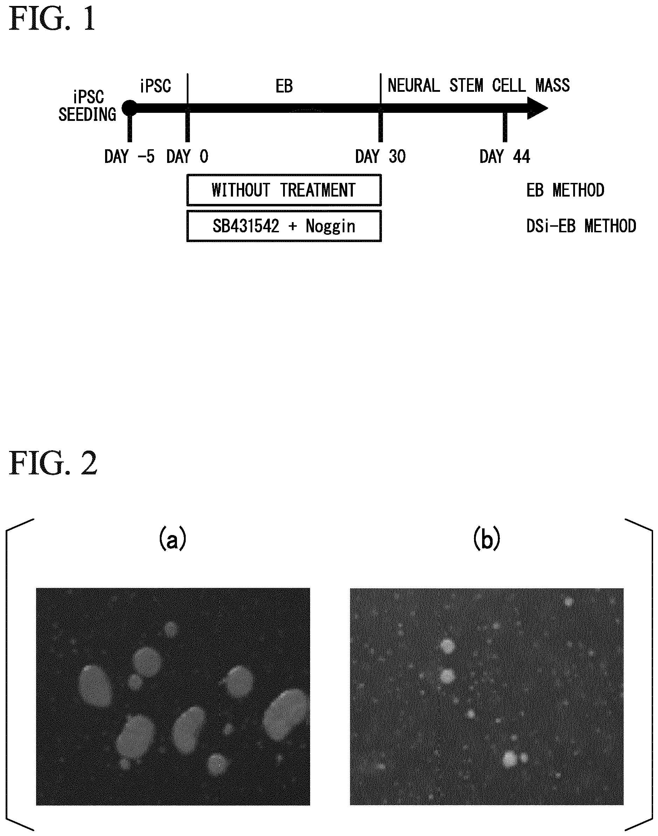

The EB method and the DSi-EB method are methods for differentiating iPSCs into neural stem cells through the formation of an embryoid body (EB). FIG. 1 is a diagram showing a procedure of the EB method and the DSi-EB method. In the EB method and the DSi-EB method, it takes more than 40 days to obtain a neural stem cell mass from iPSCs.

### «Induction of differentiation by EB method»

In the EB method, first, each iPSC dissociated into one cell was cultured to form EBs. Subsequently, EBs were differentiated into neural stem cells/neural precursor cells.

As a result of culturing each iPSC dissociated into one cell in suspension, a similar number of EBs were formed in all aHDF-iPSCs and TiPSCs. Subsequently, each EB was dissociated into one cell and cultured in a serum-free culture medium containing FGF-2 to form a neural stem cell mass (neurosphere).

As a result, all of the TiPSC strains did not form any neural stem cell masses. In contrast, all of the aHDF-iPSC strains formed neural stem cell masses within 12 days. FIGS. 2(a) and 2(b) are optical micrographs of the formed neural stem cell mass. FIG. 2(a) is a representative photograph of a neural stem cell mass formed from aHDF-iPSC. FIG. 2(b) is a representative photograph of a neural stem cell mass formed from TiPSC.

### «Induction of differentiation by DSi-EB method»

Subsequently, the examination by the DSi-EB method to form EBs in the presence of SB431542 and Noggin, which are inhibitors of the SMAD signaling pathway, was carried out. SB431542 and Noggin are known to induce dual SMAD inhibition (DSi) (see, for example, Chambers S.M., et al., Highly efficient neural conversion of human ES and iPS cells by dual inhibition of SMAD signaling, Nat. Biotechnol., 27 (3), 275-280, 2009).

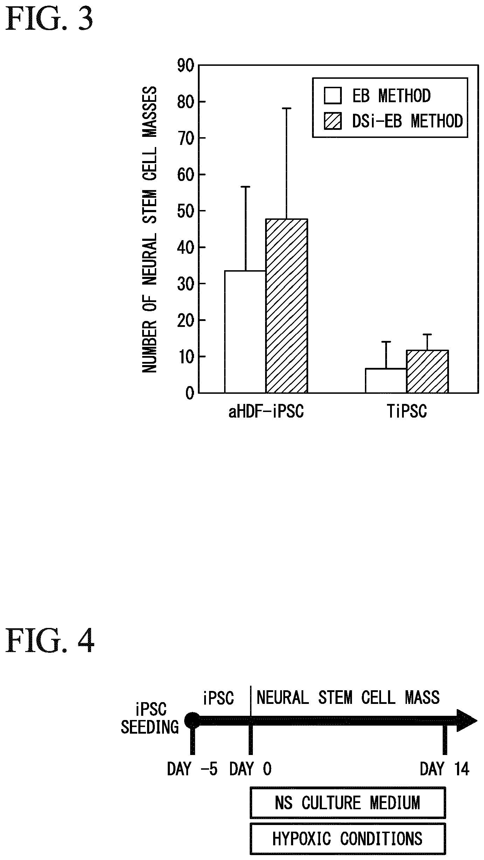

FIG. 3 is a graph showing the results of the measurement of the number of neural stem cell masses formed. As a result, as compared with the EB method, the DSi-EB method increased the number of neural stem cell masses formed from aHDF-iPSCs. However, it was not a significant difference because of the large variation from cell line to cell line. In contrast, the TiPSC strains did not form any neural stem cell masses even using the DSi-EB method.

### <TiPSC differentiated into the nervous system with the same efficiency as aHDF-iPSC, by a method of directly forming neural stem cell mass>

### [Experimental Example 3]

The present inventors have developed a novel method that does not involve the formation of EBs in the differentiation of iPSCs into neural stem cells (direct-neurosphere method, hereinafter referred to as "dNS method"). FIG. 4 is a diagram showing a procedure of the dNS method. In the dNS method, iPSCs dissociated into individual cells one by one are cultured in a serum-free culture medium (hereinafter, sometimes referred to as "NS culture medium") containing FGF2, Y27632, and LIF under hypoxic conditions. As will be described later, according to the dNS method, neural stem cell masses can be formed from iPSCs in about 14 days. The composition of a specific NS culture medium is shown in Table 1 below.

**[Table 1]**

| Composition of NS culture medium |
|---|
| 1:1 mixture of DMEM culture medium and F-12 culture medium |
| 0.6% glucose |
| 2 mM glutamine |
| 3 mM sodium bicarbonate |
| 5 mM HEPES |
| 25 µg/mL insulin |
| 100 µg/mL transferrin |
| 20 nM progesterone |
| 30 nM selenium chloride |
| 60 µM putrescine |
| 2% B27 supplement (available from Thermo Fisher Scientific Inc.) |
| 20 ng/mL FGF-2 |
| 10 µM Y-27632 (available from Wako Pure Chemical Industries, Ltd.) |
| 10 ng/mL hLIF |

The present inventors differentiated plural types of aHDF-iPSC strains and plural types of TiPSC strains into neural stem cells by the dNS method. The hypoxic conditions were 4% oxygen conditions. As the aHDF-iPSC strain, the above-mentioned KA11, KA23, eKA3, and eKA4 were used. As the TiPSC strain, the above-mentioned eTKA4, eTKA5, TKA7, TKA14, TKA4, and TKA9 were used.

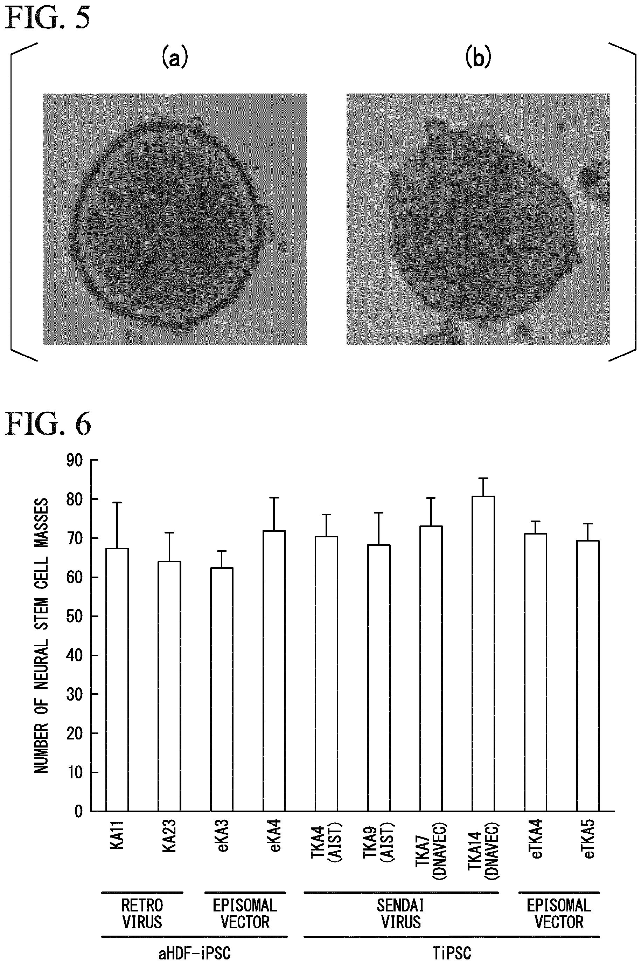

As a result, it was demonstrated that, in the dNS method, a large number of neural stem cell masses can be formed from the TiPSC strains. FIGS. 5(a) and 5(b) are optical micrographs of the formed neural stem cell mass. FIG. 5(a) is a representative photograph of a neural stem cell mass formed from aHDF-iPSC. FIG. 5(b) is a representative photograph of a neural stem cell mass formed from TiPSC.

In addition, FIG. 6 is a graph showing the results of the measurement of the number of neural stem cell masses formed. As shown in FIG. 6, all TiPSC strains and all aHDF-iPSC strains formed a similar number of neural stem cell masses.

Subsequently, the characteristics of TiPSC-derived neural stem cell mass and aHDF-iPSC-derived neural stem cell mass produced by the dNS method were examined by immunochemical analysis. As a result, it was demonstrated that both the TiPSC-derived neural stem cell mass and the aHDF-iPSC-derived neural stem cell mass expressed the forebrain marker forkhead box G1 (FOXG1) and the forebrain/midbrain marker orthodenticle homeobox 2 (OTX2).

However, these neural stem cell masses almost did not express grailed homeobox 1 (EN1), which is a marker expressed in the midbrain and hindbrain.

In addition, these neural stem cell masses also showed little expression of homeobox B4 (HOXB 4), which is a marker expressed in the myelencephalon and spinal cord. That is, it can be said that the neural stem cell mass produced by the dNS method does not substantially express HOXB4.

The above results indicate that both TiPSC and aHDF-iPSC differentiated into neural stem cell masses in the anterior region around the forebrain/midbrain in the same manner by the dNS method.

### <Examination of effect of NS culture medium and hypoxic conditions by dNS method>

### [Experimental Example 4]

In the dNS method, plural types of aHDF-iPSC strains and plural types of TiPSC strains were differentiated into neural stem cells in the presence or absence of NS culture medium and in the presence or absence of hypoxic conditions, and the effect of NS culture medium and hypoxic conditions in the dNS method was examined.

As the aHDF-iPSC strain, the above-mentioned KA11, KA23, eKA3, and eKA4 were used. As the TiPSC strain, the above-mentioned eTKA4, eTKA5, TKA7, TKA14, TKA4, and TKA9 were used.

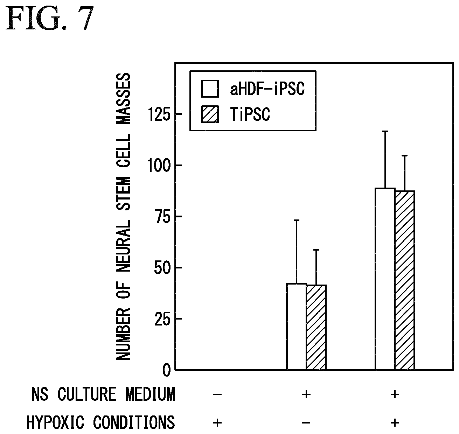

FIG. 7 is a graph showing the results of the measurement of the number of neural stem cell masses formed. In FIG. 7, the NS culture medium "+" indicates that the NS culture medium used in Experimental Example 3 was used. In addition, the NS culture medium "-" indicates that a culture medium excluding only the ROCK inhibitor (Y-27632) from the NS culture medium used in Experimental Example 3 was used. In addition, the hypoxic condition "+" indicates that the cells were cultured under 4% oxygen conditions. In addition, the hypoxic condition "-" indicates that the cells were cultured under normal oxygen conditions (20% oxygen conditions).

As a result, it was demonstrated that the NS culture medium is indispensable for the formation of neural stem cell mass by the dNS method. In addition, it was demonstrated that the number of neural stem cell masses formed is significantly increased by culturing the cells under hypoxic conditions, although the hypoxic conditions are not essential.

### <TiPSC could be differentiated into functional neuron and neuronal subtype by dNS method to the same extent as aHDF-iPSC>

### [Experimental Example 5]

Subsequently, the present inventors examined whether TiPSC-derived neural stem cell masses can be differentiated into neurons and neuronal subtypes that are as functional as aHDF-iPSC-derived neural stem cell masses.

Specifically, the neural stem cell masses were seeded on a fibronectin- and poly-L-ornithine-coated plate and cultured in a neural differentiation-inducing culture medium for about 70 days. The composition of the neural differentiation-inducing culture medium is shown in Table 2 below.

**[Table 2]**

| Composition of neural differentiation-inducing culture medium |
|---|
| 1:1 mixture of DMEM culture medium and F-12 culture medium |
| 0.6% glucose |
| 2 mM glutamine |
| 3 mM sodium bicarbonate |
| 5 mM HEPES |
| 25 µg/mL insulin |
| 100 µg/mL transferrin |
| 20 nM progesterone |
| 30 nM selenium chloride |
| 60 µM putrescine |
| 2% B27 supplement (available from Thermo Fisher Scientific Inc.) |
| 10 ng/mL brain-derived neurotrophic factor (BDNF, available from R&D Systems, Inc.) |
| 10 ng/mL glial cell-derived neurotrophic factor (GDNF, available from R&D Systems, Inc.) |
| 200 µM ascorbic acid |
| 1 mM dibutyryl-cAMP |

After 60 days, the differentiated cells were subjected to fluorescent immunostaining with antibodies against βIII-tubulin (Tuj1), which is a neuronal marker, microtubule-associated protein 2 (MAP2), which is another neuronal marker, and glial fibrillary acidic protein (GFAP), which is an astrocyte marker.

FIG. 8(a) is a representative photograph showing the results of the differentiation of the TiPSC-derived neural stem cell mass, followed by fluorescent immunostaining with an antibody (available from DAKO Corporation) against GFAP, which is an astrocyte marker. Scale bar indicates 50 µm. FIG. 8(b) is a photograph showing the results of further staining the cells in the same field of view as in FIG. 8(a) with an antibody (available from Sigma-Aldrich, Inc.) against Tuj1, which is a neuronal marker, and Hoechst 33342 (indicated as "Ho" in the figure). Hoechst 33342 is a reagent for staining the nucleus. FIG. 8(c) is a graph showing the results of the measurement of the ratio of anti-Tuj1 antibody-stained cells to Hoechst 33342-stained cells, in cells differentiated from the TiPSC-derived neural stem cell mass and cells differentiated from the aHDF-iPSC-derived neural stem cell mass. FIG. 8(d) is a graph showing the results of the measurement of the ratio of anti-GFAP antibody-stained cells to Hoechst 33342-stained cells, in cells differentiated from the TiPSC-derived neural stem cell mass and cells differentiated from the aHDF-iPSC-derived neural stem cell mass.

As a result, the proportion of cells expressing these markers was comparable between the TiPSC-derived neural stem cell mass and the aHDF-iPSC-derived neural stem cell mass. From these results, it was demonstrated that these cells differentiated into neurons and astrocytes to the same extent.

FIG. 9(a) is a representative photograph showing the results of the differentiation of the TiPSC-derived neural stem cell mass, followed by fluorescent immunostaining with an antibody (available from Sigma-Aldrich, Inc.) against MAP2, which is a neuronal marker. Scale bar indicates 50 µm. FIG. 9(b) is a photograph showing the results of further staining the cells in the same field of view as in FIG. 9(a) with an antibody (available from Sigma-Aldrich, Inc.) against Tuj1, which is another neuronal marker, and Hoechst 33342. FIG. 9(c) is a graph showing the results of the measurement of the ratio of anti-MAP2 antibody-stained cells to anti-Tuj 1 antibody-stained cells, in cells differentiated from the TiPSC-derived neural stem cell mass and cells differentiated from the aHDF-iPSC-derived neural stem cell mass.

As a result, it was demonstrated that the proportion of cells expressing these markers was comparable between the TiPSC-derived neural stem cell mass and the aHDF-iPSC-derived neural stem cell mass.

FIG. 10(a) is a representative photograph showing the results of the differentiation of the TiPSC-derived neural stem cell mass, followed by fluorescent immunostaining with an antibody (available from Millipore Corporation) against tyrosine hydroxylase (TH), which is a dopaminergic neuronal marker. Scale bar indicates 50 µm. FIG. 10(b) is a photograph showing the results of further staining the cells in the same field of view as in FIG. 10(a) with an antibody (available from Sigma-Aldrich, Inc.) against MAP2, which is a neuronal marker, and Hoechst 33342. FIG. 10(c) is a graph showing the results of the measurement of the ratio of anti-TH antibody-stained cells to anti-MAP2 antibody-stained cells, in cells differentiated from the TiPSC-derived neural stem cell mass and cells differentiated from the aHDF-iPSC-derived neural stem cell mass.

As a result, it was demonstrated that the proportion of cells differentiated into dopaminergic neurons was comparable between the TiPSC-derived neural stem cell mass and the aHDF-iPSC-derived neural stem cell mass.

FIG. 11(a) is a representative photograph showing the results of the differentiation of the TiPSC-derived neural stem cell mass, followed by fluorescent immunostaining with an antibody (available from Sigma-Aldrich, Inc.) against γ-aminobutyric acid (GABA). Scale bar indicates 50 µm. FIG. 11(b) is a photograph showing the results of further staining the cells in the same field of view as in FIG. 11(a) with an antibody (available from Sigma-Aldrich, Inc.) against MAP2, which is a neuronal marker, and Hoechst 33342. FIG. 11(c) is a graph showing the results of the measurement of the ratio of anti-GABA antibody-stained cells to anti-MAP2 antibody-stained cells, in cells differentiated from the TiPSC-derived neural stem cell mass and cells differentiated from the aHDF-iPSC-derived neural stem cell mass.

As a result, it was demonstrated that the proportion of cells differentiated into GABAergic neurons was comparable between the TiPSC-derived neural stem cell mass and the aHDF-iPSC-derived neural stem cell mass.

FIG. 12(a) is a representative photograph showing the results of the differentiation of the TiPSC-derived neural stem cell mass, followed by staining with an anti-synaptophysin antibody (available from Sigma-Aldrich, Inc.), an anti-MAP2 antibody (available from Sigma-Aldrich, Inc.), and Hoechst 33342. Scale bar indicates 50 µm. FIG. 12(b) is a photograph showing the results of staining the cells in the same field of view as in FIG. 12(a) with an antibody (Synaptics Systems Limited) against vesicular glutamate transporter 1 (vGLUT1), an anti-MAP2 antibody (available from Sigma-Aldrich, Inc.), and Hoechst 33342. FIG. 12(c) is a graph showing the results of the measurement of the ratio of anti-vGLUT1 antibody-stained cells to anti-MAP2 antibody-stained cells, in cells differentiated from the TiPSC-derived neural stem cell mass and cells differentiated from the aHDF-iPSC-derived neural stem cell mass.

As a result, it was demonstrated that the proportion of cells differentiated into glutamatergic neurons expressing synaptophysin was comparable between the TiPSC-derived neural stem cell mass and the aHDF-iPSC-derived neural stem cell mass.

From these results, it was demonstrated that TiPSC-derived neural stem cell masses can differentiate into neurons and neuronal subtypes that are as functional as aHDF-iPSC-derived neural stem cell masses.

### Industrial Applicability

According to the present invention, it is possible to provide a technique for efficiently inducing the differentiation of pluripotent stem cells into neural stem cells.

## Claims

1. A method for uniformly differentiating a pluripotent stem cell into a neural stem cell, with elimination of variation among cell strains or clones of the pluripotent stem cell and without formation of an embryoid body, regardless of the origin of the pluripotent stem cell.

2. The method according to Claim 1, comprising:
culturing the pluripotent stem cell in a culture medium containing a fibroblast growth factor 2 (FGF 2), a rho-associated protein kinase (ROCK) inhibitor, and a leukemia inhibitory factor (LIF) as active components.

3. The method according to Claim 2, wherein the culturing is carried out under hypoxic conditions.

4. The method according to Claim 2 or 3, further comprising:
dissociating the pluripotent stem cells into individual cells one by one before the culturing.

5. The method according to any one of Claims 1 to 4, wherein the pluripotent stem cell is a blood cell-derived induced pluripotent stem cell.

6. The method according to Claim 5, wherein the induced pluripotent stem cell is a T cell-derived cell.

7. A culture medium containing FGF2, a ROCK inhibitor, and LIF as active components, for use in the method according to any one of Claims 1 to 6.

8. A neural stem cell produced by the method according to any one of Claims 1 to 6.

9. The neural stem cell according to Claim 8, which expresses a forebrain marker and a forebrain/midbrain marker.

10. The neural stem cell according to Claim 8 or 9, which does not substantially express homeobox B4 (HOXB 4).

11. The neural stem cell according to any one of Claims 8 to 10, wherein a T cell receptor gene is rearranged.

12. A method for improving the efficiency of differentiating a pluripotent stem cell into a neural stem cell, the method comprising:
culturing the pluripotent stem cell in a culture medium containing FGF2, a ROCK inhibitor, and LIF as active components.

13. The method according to Claim 12, wherein the improved efficiency of differentiating a pluripotent stem cell into a neural stem cell is the efficiency equivalent to that of differentiating a dermal fibroblast-derived induced pluripotent stem cell into a neural stem cell.

14. The method according to Claim 12 or 13, wherein the culturing is carried out under hypoxic conditions.

15. The method according to any one of Claims 12 to 14, further comprising:
dissociating the pluripotent stem cells into individual cells one by one before the culturing.

16. The method according to any one of Claims 12 to 15, wherein the pluripotent stem cell is a blood cell-derived induced pluripotent stem cell.

17. The method according to Claim 16, wherein the induced pluripotent stem cell is a T cell-derived cell.

18. A culture medium containing FGF2, a ROCK inhibitor, and LIF as active components, for use in the method according to any one of Claims 12 to 17.
